# FASCICULE DE BREVET EUROPEEN

(11) **EP 3 014 160 B1**
(45) Date de publication et mention de la délivrance du brevet: **08.05.2019**
(21) Numéro de dépôt: 14749886.9
(22) Date de dépôt: 26.06.2014
(51) Int. Cl.: F16L 33/22, F16L 37/098, A61M 39/10, A61M 39/12, A61J 1/14

(54) **CONNECTEUR FLUIDIQUE AVEC SERRAGE DE TUBE INTÉGRÉ**
FLUIDSTECKER MIT INTEGRIERTER ROHRKLEMMUNG
FLUID CONNECTOR HAVING INTEGRATED PIPE CLAMPING

(30) Priorité: 28.06.2013 FR 1356355
(43) Date de publication de la demande: 04.05.2016
(73) Titulaire: SARTORIUS STEDIM FMT SAS, 13400 Aubagne (FR)
(72) Inventeur: BLAKE, Florian, F-83400 Hyeres (FR); GIBELIN, Jérémy, F-83330 Le Beausset (FR); GAY, Isabelle, F-13124 Peypin (FR)
(74) Mandataire: Derambure Conseil
(86) Numéro de dépôt international: PCT/FR2014/051623
(87) Numéro de publication internationale: WO 2014/207392

(56) Documents cités:
- EP-A2- 0 091 773
- WO-A1-2005/073613
- DE-A1- 10 355 463
- DE-B3-102005 007 217
- DE-U1- 8 610 238
- FR-A1- 2 605 709

## Description

L'invention concerne les connexions fluidiques, en particulier les connexions ou raccordements fluidiques permettant de raccorder une conduite fluide à une autre conduite ou à un récipient, dans le domaine des applications biopharmaceutiques.

Plus précisément, les conduites ou tubes utilisés dans le domaine biopharmaceutique sont des tubes souples, voire très souples, qui servent à transporter des substances biopharmaceutiques diverses, avec le plus souvent des précautions d'asepsie nécessaires. Dans les applications biopharmaceutiques, ce type de tube souple permet la circulation, le passage, la communication d'un fluide, tel qu'un fluide biopharmaceutique et peut être raccordé soit à un tube souple similaire soit à un récipient ou un contenant. Ledit récipient ou un contenant peut être rigide ou souple, ce peut être une poche, mais aussi un filtre ou une cartouche filtrante.

Un tube tel que celui auquel s'applique l'invention, habituellement de section circulaire, est réalisé typiquement en matière plastique telle que le silicone, les élastomères thermoplastiques (TPE), mais également le PVC, la liste n'étant pas limitative. Il présente une certaine tenue d'ensemble et, simultanément, à la fois une certaine flexibilité d'ensemble et une certaine flexibilité locale, ce qui permet, moyennant l'exercice d'une force suffisante, de pincer le tube ou de le déformer substantiellement radialement.

Dans une réalisation typique, par exemple, le tube a un diamètre extérieur compris entre par exemple 8 millimètres et 30 millimètres, l'épaisseur dépendant du matériau, du diamètre et des applications.

Dans l'art connu, pour accoupler un tel tube souple, on enfile celui-ci sur un embout tubulaire, après quoi on place un collier de serrage autour du tube, puis on procède au serrage du collier. Ainsi, le collier serré exerce une pression radiale vers l'intérieur pour maintenir le tube souple sur l'embout, d'une part pour assurer une bonne étanchéité du tube vis-à-vis de l'embout et d'autre part pour éviter qu'une traction sur le tube ne conduise à un désengagement du tube de l'embout.

S'agissant de tels colliers de serrage, on peut utiliser par exemple un collier en matière plastique, de type polyamide par exemple en Rilsan®. Ce type de collier plastique, aussi parfois appelé Serflex®, comprend un système de crans sur une bande qui coopèrent avec un crochet à verrouillage agencé dans la tête, de manière à ce que le serrage ne soit pas réversible. En d'autres termes, une fois que l'on a engagé la bande dans la tête pour former une boucle, on tire sur la bande pour réduire le diamètre de la boucle et serrer le collier, le retour en arrière étant empêché par le crochet à verrouillage en prise dans un des crans de la bande. Après serrage, pour éviter que la bande ne dépasse trop du diamètre de la boucle du collier, on sectionne la portion libre de bande à proximité de la tête du collier. La partie restante non détachée de la bande présente souvent une arête vive qui peut s'avérer tranchante.

En alternative au collier plastique, on peut aussi utiliser un collier métallique qui se présente sous la forme d'un anneau préformé muni d'une ou deux oreilles faisant saillie vers l'extérieur relativement à la forme générale de l'anneau du collier, ce type de collier est parfois appelé collier Oetiker®. Après insertion du collier sur le tube à maintenir, on procède au moyen d'un outil au pincement de l'oreille (ou des oreilles) du collier ce qui provoque une déformation rémanente et ainsi un rétrécissement du diamètre principal de l'anneau et par conséquent serrage du collier sur le tube. Ce type de serrage par anneau métallique est particulièrement robuste et fiable. Toutefois, à l'endroit de l'oreille pincée par l'outil, il peut se présenter une aspérité ou une bavure qui forme une arête vive qui peut s'avérer agressive.
Il faut toutefois noter qu'il est aussi possible d'utiliser des colliers métalliques à rétreint serti, sans oreille.

Lorsque que de tels colliers, qu'ils soient plastiques ou métalliques, sont installés dans des ensembles biopharmaceutiques, ces derniers peuvent être amenés à être transportés ou déplacés, et par conséquent il existe un risque d'agression par des parties agressives de ces colliers vis-à-vis d'autres éléments de l'ensemble biopharmaceutique, en particulier des poches souples ou des tubes souples, ce qui peut entraîner une fuite ou une perte de stérilisation préjudiciable à l'application biopharmaceutique.
De plus, ces colliers sont faciles d'accès (et donc peuvent être démontés) et ne permettent pas de garantir une image et une esthétique satisfaisantes.

De plus, les solutions précitées à base de collier sont imparfaites en ce qui concerne l'étanchéité entre le tube souple et l'embout tubulaire. En effet, l'étanchéité au niveau de l'oreille du collier métallique ou de la tête du collier plastique présente une discontinuité de pression radiale, et par conséquent elle est perfectible.

En outre, le serrage de tels colliers est effectué par une opération manuelle ; la fiabilité d'une telle opération est moins élevée que celle d'une opération automatisée.

Le document DE 86 10 238 U1 divulgue un connecteur électrique pour raccorder un tube ondulé de protection électrique à une connexion d'un appareil.

Le document DE 103 55 463 A1 divulgue un raccord utilisé dans le domaine automobile.

Le document DE 10 2005 007 217 B3 divulgue un raccord d'un tube ondulé. Le raccord comprend deux pattes flexibles situées diamétralement opposées d'un embout.

Le document FR 2 605 709 A1 divulgue un raccord pour des embouts de boîte à eau d'échangeur de chaleur. Des pattes flexibles s'étendent au repos selon un cylindre.

Il existe par conséquent le besoin de proposer une amélioration destinée à pallier, au moins en partie, un des inconvénients précités de l'art antérieur connu.

Ci-après, un exposé de l'invention telle que caractérisée dans les revendications.

Selon un premier aspect, l'invention a pour objet l'utilisation dans le domaine biopharmaceutique d'un ensemble biopharmaceutique selon la revendication 1.

Moyennant quoi le serrage du tube est particulièrement performant. En particulier, le serrage est bien réparti et homogène sur toute la circonférence autour du tube et sur une certaine longueur axiale. En outre, il n'est pas nécessaire de tourner la bague, un mouvement de déplacement axial suffit et la bonne position active peut être visuellement aisément vérifiée.

De plus, on peut envisager une automatisation de l'insertion du tube et de la bague pour former un dispositif de raccord avec la fiabilité d'une opération automatisée.

Il n'est toutefois pas est exclu d'assembler manuellement le dispositif de raccord, et dans ce cas ceci peut être fait avantageusement sans outil.

Selon l'invention, les pattes flexibles sont venues de matière depuis la zone intermédiaire du corps et s'étendent dans leur état au repos selon un cône qui s'ouvre en direction de l'extrémité de l'embout, moyennant quoi l'extrémité du tube souple peut être insérée en début d'assemblage sur l'embout sans que les pattes flexibles ne présentent un obstacle. En outre, ceci présente l'avantage de faciliter le démoulage lors de la fabrication du connecteur.

Selon une réalisation, lequel les extrémités libres des pattes flexibles comprennent des chanfreins d'accostage pour la bague. De sorte qu'au début du mouvement d'insertion de la bague, cette dernière agit sur les chanfreins de manière à presser les pattes flexibles radialement vers l'intérieur.

Selon une réalisation, la bague comprend une zone évasée conique pour accoster par l'extérieur les extrémités libres des pattes flexibles. Moyennant quoi, l'effort d'insertion exercé sur le bague par un opérateur ou un outil de machine permet d'exercer un effort comprenant une composante radiale vers l'intérieur de manière à resserrer les pattes flexibles.

Selon une réalisation, le connecteur comprend une collerette et la collerette forme une butée pour la fin du mouvement d'insertion axiale de la bague ; ce qui forme un repère simple et fiable pour la fin du mouvement d'insertion.

Selon une réalisation, les pattes flexibles comprennent au moins une dent sur la face radialement intérieure, de sorte que la pression sur le tube est plus importante en vis-à-vis de cette dent ; qui constitue un ancrage destiné à éviter un retrait du tube même en cas de traction sur le tube.

Selon une réalisation, l'embout comprend au moins un cran décalé axialement par rapport à la dent; ce qui permet de renforcer l'effet d'ancrage précité du tube sur l'embout.

Selon une réalisation, en position active de la bague, les pattes sont prises en sandwich entre la bague et le tube sur une longueur représentant plus de la moitié de la longueur axiale de la bague, et de préférence sur une longueur supérieure au diamètre intérieur de l'embout. On obtient ainsi une surface de contact importante entre le tube et l'embout, ce qui favorise le maintien et étanchéité.

Selon une réalisation, en position active de la bague, les pattes flexibles sont adjacentes les unes aux autres selon la direction circonférentielle. De sorte que la pression radiale des pattes flexibles s'exerce sur la totalité du pourtour du tube.

Selon une réalisation, le premier connecteur forme une interface mâle apte à être reçue dans un second connecteur formant une interface femelle ; ce qui représente une solution d'interface fluidique particulièrement simple, pour raccorder plusieurs types de tube à l'entité solidaire du second connecteur.

Le premier connecteur comprend un disque de fixation configuré pour être fixé directement à un contenant de type poche ou filtre ; ce qui permet de raccorder facilement le tube directement à un contenant.

Selon une réalisation, la bague comprend un épaulement radial extérieur destiné à faciliter le mouvement de déplacement depuis la position d'attente vers la position active ; ce qui autorise une automatisation de l'insertion de la bague ; ceci permet aussi d'envisager un déplacement de la bague sans outil et par conséquent un assemblage du raccord complètement manuel.

Selon une réalisation, la bague comprend une nervure annulaire reçue dans une encoche ménagée sur la face extérieure de chaque patte flexible ; moyennant quoi on obtient une solution de verrouillage de la position de la bague dans sa position active, peu encombrante radialement et axialement.

Selon une réalisation, la nervure annulaire présente une section avec une pointe dirigée vers l'arrière du tube ; ce qui procure une forme de harpon particulièrement efficace pour le verrouillage de la position active.

Selon une réalisation, lequel la bague comprend un clip de fixation configuré pour assurer le clipsage de la bague sur la collerette dans la position active ; ce qui représente une solution alternative pour le maintien axial de la bague dans sa position active.

Selon une réalisation, le premier connecteur ou la bague peut comprendre en outre un identifiant de type code barre ou étiquette RFID ou code couleur. Moyennant quoi l'accès à des informations relatives à la poche souple et/ou au produit biopharmaceutique y contenu est aisé, en particulier pour contribuer à la fonction traçabilité.

Selon un deuxième aspect, l'invention a pour objet une méthode pour former un dispositif de raccord destiné à raccorder de manière étanche un tube souple avec une paroi lisse pour fluide biopharmaceutique à un embout tubulaire d'un connecteur selon la revendication 16.

Moyennant quoi, on forme le dispositif de raccord par un mouvement axial uniquement de la bague, ce que l'on peut obtenir par une opération manuelle sans outil ou par une opération automatisée.

On décrit maintenant brièvement les figures des dessins.
La figure 1 est une vue éclatée d'un dispositif de raccord conforme à la présente invention.
La figure 2 est une vue en coupe axiale du dispositif de raccord de la figure 1, en position séparée.
La figure 3 est une coupe axiale plus détaillée du dispositif de raccord de la figure 1, en position accouplée.
La figure 4 est analogue à la figure 1 et montre une variante du dispositif de raccord.

Ci-après un exposé détaillé d'un mode de réalisation de l'invention assorti d'exemples et de référence aux dessins.

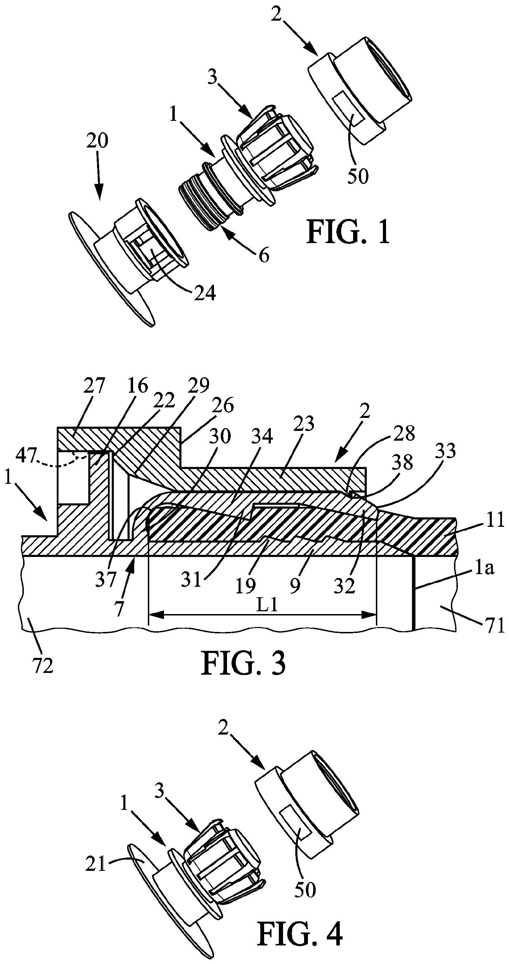

Dans l'exemple illustré aux figures 1-3, il s'agit de raccorder un tube souple **11** à un premier connecteur **1**, de façon étanche, au moyen d'une bague distincte **2** qui sera décrite plus loin. Le premier connecteur est destiné à être accouplé à un second connecteur **20** pour former une connexion fluide entre deux espaces fluidiques.

Le tube souple 11 peut être défini généralement comme une première paroi 11 délimitant un premier espace fluidique **71.** Le diamètre extérieur du tube souple 11 au repos est référencé D2.

Le premier connecteur **1** est réalisé en matière synthétique, plus précisément il peut être obtenu par moulage d'une matière plastique, par exemple polypropylène, du polyéthylène, du polycarbonate, du polysulfone.
Le premier connecteur 1 comprend un embout tubulaire **9** du côté de l'une de ses extrémités 1a, et une interface de couplage **6** avec un second connecteur **20** à l'autre de ses extrémités. Le premier connecteur 1 comprend en outre une portion intermédiaire **7** qui sera détaillée plus loin.

L'embout tubulaire **9** est de révolution autour de l'axe A et forme une deuxième paroi. On peut remarquer que le diamètre intérieur D1 de l'embout tubulaire 9 est sensiblement voisin du diamètre intérieur du tube souple 11 au repos, mais il faut noter que l'embout est prévu pour recevoir une gamme de tubes de diamètre sensiblement différent autour de D1, ledit tube se déformant aisément radialement au moment de l'insertion.
Lorsque l'on enfile le tube souple **11** sur l'embout tubulaire 9, le tube se déforme radialement vers l'extérieur grâce à la forme de rampe formée par le chanfrein d'entrée, puis au fur et à mesure de l'insertion vers l'avant selon la direction de la flèche **F**, il se conforme au diamètre extérieur de l'embout.
L'insertion du tube souple peut se poursuivre jusqu'à ce que l'extrémité avant du tube **11a** vienne porter contre une zone d'arrêt **30** qui sera décrite plus loin (voir figure 3).

L'embout **9** peut présenter une surface externe sous forme de cylindre lisse, il peut comprendre ou pas un ou plusieurs crans annulaires **19**, qui dans l'exemple illustré présentent chacun une rampe douce du côté du tube souple à insérer et un épaulement du côté opposé. L'embout tubulaire pourrait comprendre un nombre différent de crans voire un seul cran ou pas de cran.

Dans l'exemple illustré, l'interface de couplage **6** est une interface de type mâle qui est prévue pour être insérée dans une interface de type femelle du second connecteur 20. Mais bien sûr, le contraire serait possible, c'est-à-dire une interface mâle dans le second connecteur et une interface femelle dans le premier connecteur, voire encore une interface asexuée.

Dans l'exemple présenté, l'interface mâle **6** dans le premier connecteur est généralement cylindrique de révolution autour de l'axe A, avec une ou deux rainures extérieures destinées à recevoir un ou deux joints toriques **35** pour assurer la fonction étanchéité.

La portion intermédiaire **7** comprend une première collerette **16** en forme de disque, de diamètre plus grand que D2, cette première collerette sert de butée d'arrêt pour l'insertion de la bague, et une deuxième collerette **14** également en forme de disque, mais plus petit, dont l'utilité sera vue plus loin.

Avantageusement, le premier connecteur 1 comprend une pluralité de pattes flexibles **3**, en nombre de 4 à 16, réparties tout autour de la circonférence de l'embout. Chacune des pattes flexibles **3** comprend une emplanture **30**, qui s'étend à partir de la portion intermédiaire 7 au voisinage de la base de première collerette **16.** Cette emplanture **30** forme une butée pour l'extrémité avant 11a du tube lorsque l'on insère ce dernier sur l'embout 9. Chaque patte flexible 3 comprend en outre une branche principale **34** reliée par un coude souple **37** à l'emplanture **30**, ladite branche principale s'étendant jusqu'à une extrémité libre **32.** La longueur **L1** de la branche principale 34 est supérieure au diamètre intérieur **D1** du tube, et peut même être supérieur diamètre extérieur **D2** du tube.

Sur la face intérieure de la branche principale, chaque patte flexible comprend au moins une dent **31** destinée à venir s'enfoncer dans la surface extérieure du tube comme il sera précisé plus loin. Il pourrait y avoir plus d'une dent, par exemple un autre au voisinage de l'extrémité **32.**
La pénétration de la dent est substantielle car la matière du tube souple est moins dure que la matière des pattes flexibles.

L'extrémité libre **32** comprend sur la face extérieure un chanfrein **33** pour l'accostage de la bague 2 et une rainure **38** dont l'utilité sera vue plus loin.

La bague distincte **2** se présente comme une pièce annulaire de révolution en matière plastique plus dure que celle du premier connecteur, par exemple en polyamide, PBT, ABS, ou autre matière plastique analogue. La bague 2 comprend une première portion annulaire **23** de diamètre intérieur **D3**, et une seconde portion annulaire **27** de diamètre supérieur dirigé vers l'avant du connecteur. Une zone conique évasée **29** est disposée radialement intérieurement dans la seconde portion cylindrique au voisinage de son raccordement avec la première portion cylindrique. En outre, un épaulement **26** est agencé radialement extérieurement entre la première portion cylindrique et la seconde portion cylindrique. C'est épaulement 26 peut servir de surface de portée pour l'intervention d'un outil d'automatisation ; il sert aussi de surface de préhension lorsque la bague est insérée manuellement par les doigts d'un utilisateur.

La bague 2 est disposée en attente (dans une position d'attente) en arrière sur le tube préalablement à son assemblage sur le premier connecteur 1.

La méthode pour assembler un tel dispositif de raccord est présenté ci-dessous :
**a/** on fournit un connecteur 1 en plastique moulé, ledit connecteur comprenant l'embout tubulaire 9 précité, prévu pour recevoir le tube souple précité,
**/b/** on dispose la bague distincte 2 précitée dans une position d'attente sur le tube,
**/c/** insérer le tube 11 sur l'embout tubulaire 9 selon un axe A jusqu'à la zone d'arrêt 30,
**/d/** déplacer la bague 2 depuis la position d'attente jusque dans une position active, dans laquelle elle vient buter sur la première collerette 16.

On remarque que, dans la solution illustrée, la bague 2 est déplacée en direction du corps du connecteur pour passer de la position d'attente à la position active, autrement dit la bague est déplacée de l'arrière du tube vers l'extrémité avant du tube, selon la même direction F que le mouvement d'insertion tube souple.

On décrit en détail maintenant l'étape /d/. Au cours du mouvement d'insertion de la bague **2** selon la flèche **F**, en premier lieu la zone conique évasée **29** accoste les chanfreins **33** de l'extrémité libre **32** des pattes flexibles, ce qui engendre un début de mouvement de resserrement vers le centre de l'axe des dites pattes flexibles.
Au cours de la progression du mouvement, la bague 2, en particulier sa portion annulaire 23, recouvre progressivement toute la longueur de la branche principale **34** de chaque patte flexible, de manière à recouvrir complètement les pattes flexibles 3 pour les contraindre radialement vers l'intérieur, de manière à presser le tube 11 sur l'embout 9.

Selon un aspect avantageux, dans la position active, les pattes s'étendent sensiblement parallèlement à l'axe. L'embout **9** et les pattes flexibles **3** prennent le tube 11 en sandwich sur la longueur **L1,** supérieure à D1. Dans une variante non représentée, la longueur **L1** pourrait être supérieure au diamètre extérieur du tube D2.
Avantageusement, les pattes flexibles 3 sont donc contraintes radialement vers l'intérieur de manière à presser le tube 11 sur l'embout 9 dans ladite position active, permettant ainsi de créer radialement un contact étanche entre le tube 11 et l'embout tubulaire 9.

Dans une variante non représentée, les pattes flexibles 3 pourraient être plus longues et dépasser de l'extrémité **1a** de l'embout 9.

À la fin du mouvement d'insertion axiale, un épaulement avant **22** de la bague vient buter sur la collerette **16.**
Il faut remarquer ici que le mouvement d'insertion ne nécessite pas de tourner la bague. Toutefois si la bague est tournée pendant le mouvement d'insertion cela ne change pas l'effet radial qu'elle apporte sur les pattes flexibles.

La simplicité de cette insertion manuelle ou automatisée permet d'obtenir une amélioration de la fiabilité du montage de l'interface.

Selon un aspect optionnel, dans la position active atteinte lorsque la bague 2 est en butée sur la collerette 16, une nervure annulaire **28** dirigée vers l'intérieur et de préférence vers l'arrière, est reçu par complémentarité de forme dans une encoche **38** (ou rainure) prévue sur la face extérieure arrière de la branche principale 34 de chaque patte flexible 3. L'encoche ou rainure 38 est agencée au début du chanfrein dans l'exemple illustré, mais pourrait être située ailleurs.

Selon un aspect optionnel alternatif à la solution précédente, on pourrait disposer un (ou plusieurs) clip de verrouillage **47** (ou clip de fixation) sur la partie avant de la portion annulaire **27** avant de la bague, de sorte que cela produit, en coopération avec l'épaulement avant 22 précité, un clipsage de la bague **2** sur la collerette 16 dans la position active de la bague (cf Fig. 3).

Selon un aspect optionnel, il peut être prévu des languettes de verrouillage **24** dans le corps du second connecteur **20.** Ces languettes de verrouillage 24, au nombre de deux dans l'exemple illustré sont flexibles, elles s'écartent vers l'extérieur au moment où la deuxième collerette **14** avance au niveau de leur extrémité libre, puis reviennent vers l'intérieur pour se positionner en face d'une butée anti retrait sur l'arrière de la deuxième collerette 14. Après quoi il n'est pas possible de faire marche arrière c'est-à-dire de retirer le premier connecteur du second connecteur sans au préalable effacer d'une façon ou d'une autre les languettes de verrouillage 24.

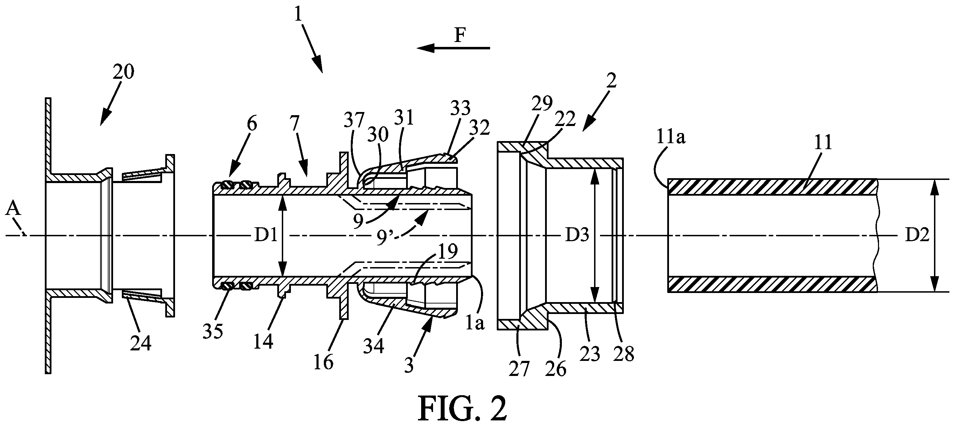

Il faut aussi remarquer que l'interface mâle 6 peut être d'une dimension standard pour plusieurs types de diamètres de tube **11** et d'embout **9,9'** (cf Fig. 2). Moyennant une diversité sur le diamètre de l'embout du premier connecteur, on peut alors interfacer plusieurs types de tube de diamètres différents sur un seul et même type de second connecteur **20.**

Dans la variante de réalisation illustrée à la figure 4, le connecteur 1 forme une embase d'interface avec une poche souple ou rigide, sans notion d'interface mâle ou femelle. Le dispositif de raccord permet alors de raccorder de façon fiable un tube souple directement à une poche, au moyen d'un disque de fixation **21** annulaire assez large qui est fixé par soudure à la poche ou à une paroi de cartouche filtrante.

En outre, il est prévu une caractéristique optionnelle compatible avec toutes les variantes de réalisation précédemment évoquées : il s'agit de l'intégration d'un identifiant 50, de type code barre ou étiquette électronique par exemple de type RFID ou encore un code couleur. De façon préférée, on dispose cet identifiant sur la bague 2, mais on pourrait dans le cas de l'étiquette RFID prévoir que celle-ci est disposée sur le premier connecteur 1, accessible par lecture de type transpondeur même après l'insertion complète de la bague 2.

Il faut noter que le second connecteur 20 peut s'interfacer avec un second tube souple ou un filtre au lieu d'avoir un disque de fixation à une poche comme illustré à la figure 1.

## Revendications

1. Utilisation dans le domaine biopharmaceutique d'un ensemble biopharmaceutique comprenant un dispositif de raccord apte et destiné à raccorder une première paroi délimitant un premier espace fluidique (71), en forme de tube souple (11), à une seconde paroi formée par un embout tubulaire (9) d'un connecteur (1), dans un ensemble biopharmaceutique, de sorte à être apte et destiné à assurer une étanchéité entre la première paroi et la seconde paroi formée par l'embout tubulaire, le dispositif de raccord comprenant :
- ledit connecteur (1) avec :
. ledit embout tubulaire (9), sur lequel peut être inséré une extrémité du tube selon un axe A, depuis une première extrémité de l'embout jusqu'à une zone d'arrêt (30) du tube,
. une pluralité de pattes flexibles (3) s'étendant depuis une zone intermédiaire (7) du corps du connecteur en direction de l'extrémité libre (1a) de l'embout, les pattes flexibles étant réparties tout autour de la circonférence de l'embout,
. une interface de connexion (6;21) disposée à l'opposé de l'embout tubulaire,
- une bague distincte (2), déplaçable axialement entre une position d'attente et une position active, apte et destinée à recouvrir les pattes flexibles (3) pour les contraindre radialement vers l'intérieur de manière à presser le tube sur l'embout dans ladite position active, permettant ainsi de créer radialement un contact étanche entre le tube et l'embout tubulaire,
les pattes s'étendant sensiblement parallèlement à l'axe (A) quand la bague (2) est dans la position active,
dans lequel la bague distincte est déplacée en direction du corps du connecteur pour passer de la position d'attente à la position active,
et dans lequel les pattes flexibles (3) sont venues de matière depuis la zone intermédiaire (7) du corps et s'étendent dans leur état au repos selon un cône qui s'ouvre en direction de l'extrémité (1a) de l'embout, moyennant quoi l'extrémité du tube souple peut être insérée sur l'embout sans que les pattes flexibles ne présentent un obstacle.

2. Utilisation dans le domaine biopharmaceutique d'un ensemble biopharmaceutique comprenant un dispositif selon la revendication 1, dans lequel les extrémités libres (32) des pattes flexibles comprennent des chanfreins d'accostage (33) pour la bague.

3. Utilisation dans le domaine biopharmaceutique d'un ensemble biopharmaceutique comprenant un dispositif selon l'une des revendications 1-2, dans lequel la bague comprend une zone évasée conique (29) pour accoster par l'extérieur les extrémités libres des pattes flexibles (3).

4. Utilisation dans le domaine biopharmaceutique d'un ensemble biopharmaceutique comprenant un dispositif selon l'une des revendications 1-3, dans lequel le connecteur comprend une collerette (16) et la collerette forme une butée pour la fin du mouvement d'insertion axiale de la bague.

5. Utilisation dans le domaine biopharmaceutique d'un ensemble biopharmaceutique comprenant un dispositif selon l'une des revendications 1-4, dans lequel les pattes flexibles comprennent au moins une dent (31) sur la face radialement intérieure, de sorte que la pression sur le tube est plus importante en vis-à-vis de cette dent.

6. Utilisation dans le domaine biopharmaceutique d'un ensemble biopharmaceutique comprenant un dispositif selon la revendication 5, dans lequel l'embout comprend un cran (19) décalé axialement par rapport à la dent (31).

7. Utilisation dans le domaine biopharmaceutique d'un ensemble biopharmaceutique comprenant un dispositif selon l'une des revendications 1-6, dans lequel en position active de la bague, les pattes (3) sont prises en sandwich entre la bague et le tube sur une longueur (L1) représentant plus de la moitié de la longueur axiale de la bague, et de préférence sur une longueur supérieure au diamètre intérieur (D1) de l'embout.

8. Utilisation dans le domaine biopharmaceutique d'un ensemble biopharmaceutique comprenant un dispositif selon l'une des revendications 1-7, dans lequel, en position active de la bague, les pattes flexibles (3) sont adjacentes les unes aux autres selon la direction circonférentielle.

9. Utilisation dans le domaine biopharmaceutique d'un ensemble biopharmaceutique comprenant un dispositif selon l'une des revendications 1-8, dans lequel le premier connecteur (1) forme une interface mâle (6) apte à être reçue dans un second connecteur (20) formant une interface femelle.

10. Utilisation dans le domaine biopharmaceutique d'un ensemble biopharmaceutique comprenant un dispositif selon l'une des revendications 1-9, dans lequel le premier connecteur comprend un disque de fixation (21) configuré pour être fixé directement à un contenant de type poche ou filtre.

11. Utilisation dans le domaine biopharmaceutique d'un ensemble biopharmaceutique comprenant un dispositif selon l'une des revendications 1-10, dans lequel la bague (2) comprend un épaulement (26) radial extérieur destiné à faciliter le mouvement de déplacement depuis la position d'attente vers la position active.

12. Utilisation dans le domaine biopharmaceutique d'un ensemble biopharmaceutique comprenant un dispositif selon l'une des revendications 1-11, dans lequel la bague (2) comprend une nervure annulaire (28) reçue dans une encoche (38) ménagée sur la face extérieure de chaque patte flexible.

13. Utilisation dans le domaine biopharmaceutique d'un ensemble biopharmaceutique comprenant un dispositif selon la revendication 12, dans lequel la nervure annulaire (28) présente une section avec une pointe dirigée vers l'arrière du tube.

14. Utilisation dans le domaine biopharmaceutique d'un ensemble biopharmaceutique comprenant un dispositif selon l'une des revendications 1-13, dans lequel la bague comprend un clip de fixation (47) configuré pour assurer le clipsage de la bague sur la collerette (16) dans la position active.

15. Utilisation dans le domaine biopharmaceutique d'un ensemble biopharmaceutique comprenant un dispositif selon l'une des revendications 1-14, dans lequel le premier connecteur ou la bague comprend en outre un identifiant (50) de type code barre ou étiquette RFID ou code couleur.

16. Méthode pour former un ensemble biopharmaceutique comprenant un dispositif de raccord destiné à raccorder de manière étanche un tube souple (11) avec une paroi lisse pour fluide biopharmaceutique à un embout tubulaire (9) d'un connecteur (1),
la méthode comprenant les étapes :
/a/ fournir un connecteur en plastique moulé, ledit connecteur comprenant:
. un embout tubulaire (9), sur lequel peut être inséré une extrémité d'un tube souple avec une paroi lisse selon un axe A, avec une zone d'arrêt (30) pour limiter la course d'insertion du tube,
. une pluralité de pattes flexibles (3) s'étendant depuis une zone intermédiaire (7) du corps du connecteur en direction de l'extrémité libre (1a) de l'embout, les pattes flexibles étant réparties tout autour de la circonférence de l'embout, et venues de matière depuis la zone intermédiaire (7) du corps, et s'étendant dans leur état au repos selon un cône qui s'ouvre en direction de l'extrémité (1a) de l'embout,
. une interface de connexion (6;21) disposée à l'opposé de l'embout tubulaire,
**/b/** fournir une bague distincte (2), et la disposer dans une position d'attente en arrière sur le tube,
**/c/** insérer le tube souple (11) avec une paroi lisse sur l'embout tubulaire jusqu'à la zone d'arrêt,
**/d/** déplacer axialement la bague depuis la position d'attente en direction du corps du connecteur jusque dans une position active laquelle est apte et destinée à recouvrir les pattes flexibles (3) pour les contraindre radialement vers l'intérieur de manière à presser le tube sur l'embout dans ladite position active les pattes s'étendant sensiblement parallèlement à l'axe (A) quand la bague (2) est dans la position active.

## Patentansprüche

1. Verwendung einer biopharmazeutischen Anordnung auf dem Gebiet der Biopharmazeutik, aufweisend eine Kopplungsvorrichtung, die geeignet und vorgesehen ist, in einer biopharmazeutischen Anordnung eine einen ersten Fluidraum (71) begrenzende erste Wand in Form eines weichen Rohrs (11) mit einer von einem rohrförmigen Stück (9) eines Verbinders (1) gebildeten zweiten Wand zu koppeln, um so geeignet und vorgesehen zu sein, eine Dichtheit zwischen der ersten Wand und der von dem rohrförmigen Stück gebildeten zweiten Wand sicherzustellen, wobei die Kopplungsvorrichtung aufweist:
- den Verbinder (1) mit:
* dem rohrförmigen Stück (9), auf das ein Ende des Rohrs gemäß einer Achse A von einem ersten Ende des Stücks bis zu einer Rohranschlagzone (30) geschoben werden kann,
* mehreren flexiblen Klauen (3), die sich von einer Mittelzone (7) des Körpers des Verbinders in Richtung freies Ende (1a) des Stücks erstrecken, wobei die flexiblen Klauen um den gesamten Umfang des Stücks verteilt sind,
* einer Verbindungsschnittstelle (6; 21), die auf der bezüglich des rohrförmigen Stücks entgegengesetzten Seite angeordnet ist,
- einen zwischen einer Warteposition und einer aktiven Position axial verschiebbaren separaten Ring (2), der geeignet und vorgesehen ist, in der aktiven Position die flexiblen Klauen (3) zu bedecken, um sie radial einwärts zu zwängen, um das Rohr an das Stück zu pressen, um so zu erlauben, radial einen dichten Kontakt zwischen dem Rohr und dem rohrförmigen Stück zu erzeugen, wobei die Klauen sich im Wesentlichen parallel zur Achse (A) erstrecken, wenn der Ring (2) in der aktiven Position ist,
wobei der separate Ring in Richtung des Körpers des Verbinders verschoben wird, um von der Warteposition in die aktive Position überzugehen,
und die flexiblen Klauen (3) integral mit der Mittelzone (7) des Körpers sind und sich in ihrer Ruheposition gemäß einem Kegel erstrecken, der sich zum Ende (1a) des Stücks hin öffnet, wodurch das Ende des weichen Rohrs auf das Stück geschoben werden kann, ohne dass die flexiblen Klauen ein Hindernis darstellen.

2. Verwendung einer biopharmazeutischen Anordnung auf dem Gebiet der Biopharmazeutik, die eine Vorrichtung nach Anspruch 1 aufweist, in welcher die freien Enden (32) der flexiblen Klauen Eingriffsschrägen (33) für den Ring aufweisen.

3. Verwendung einer biopharmazeutischen Anordnung auf dem Gebiet der Biopharmazeutik, die eine Vorrichtung nach einem der Ansprüche 1 bis 2 aufweist, in welcher der Ring eine konisch erweiterte Zone (29) aufweist, um von außen die freien Enden der flexiblen Klauen (3) zu greifen.

4. Verwendung einer biopharmazeutischen Anordnung auf dem Gebiet der Biopharmazeutik, aufweisend eine Vorrichtung nach einem der Ansprüche 1 bis 3, in welcher der Verbinder einen Kragen (16) aufweist und der Kragen einen Anschlag für das Ende der axialen Vorschubbewegung des Rings bildet.

5. Verwendung einer biopharmazeutischen Anordnung auf dem Gebiet der Biopharmazeutik, aufweisend eine Vorrichtung nach einem der Ansprüche 1 bis 4, in welcher die flexiblen Klauen mindestens einen Zahn (31) an der radial inneren Oberfläche aufweisen, derart, dass gegenüber diesem Zahn der Druck auf den Schlauch stärker ist.

6. Verwendung einer biopharmazeutischen Anordnung auf dem Gebiet der Biopharmazeutik, aufweisend eine Vorrichtung nach Anspruch 5, in welcher das Stück eine Kerbe (19) aufweist, die bezüglich des Zahns (31) axial versetzt angeordnet ist.

7. Verwendung einer biopharmazeutischen Anordnung auf dem Gebiet der Biopharmazeutik, aufweisend eine Vorrichtung nach einem der Ansprüche 1 bis 6, in welcher in der aktiven Position des Rings die Klauen (3) zwischen dem Ring und dem Rohr über eine Länge (L1) eingezwängt sind, die mehr als die Hälfte der axialen Länge des Rings ist, und vorzugsweise über eine Länge, die größer als der Innendurchmesser (D1) des Stücks ist.

8. Verwendung einer biopharmazeutischen Anordnung auf dem Gebiet der Biopharmazeutik, aufweisend eine Vorrichtung nach einem der Ansprüche 1 bis 7, in welcher in der aktiven Position des Rings die flexiblen Klauen (3) in der Umfangsrichtung aneinander angrenzend sind.

9. Verwendung einer biopharmazeutischen Anordnung auf dem Gebiet der Biopharmazeutik, aufweisend eine Vorrichtung nach einem der Ansprüche 1 bis 8, in welcher der erste Verbinder (1) eine männliche Schnittstelle (6) bildet, die geeignet ist, in einem zweiten Verbinder (20), der eine weibliche Schnittstelle bildet, aufgenommen zu werden.

10. Verwendung einer biopharmazeutischen Anordnung auf dem Gebiet der Biopharmazeutik, aufweisend eine Vorrichtung nach einem der Ansprüche 1 bis 9, in welcher der erste Verbinder eine Befestigungsscheibe (21) aufweist, die konfiguriert ist, direkt an einem Behältnis des Typs Beutel oder Filter befestigt zu werden.

11. Verwendung einer biopharmazeutischen Anordnung auf dem Gebiet der Biopharmazeutik, aufweisend eine Vorrichtung nach einem der Ansprüche 1 bis 10, in welcher der Ring (2) eine radiale äußere Schulter (26) aufweist, die vorgesehen ist, die Verschiebungsbewegung von der Warteposition zur aktiven Position zu erleichtern.

12. Verwendung einer biopharmazeutischen Anordnung auf dem Gebiet der Biopharmazeutik, aufweisend eine Vorrichtung nach einem der Ansprüche 1 bis 11, in welcher der Ring (2) eine ringförmige Rippe (28) aufweist, die in einer an der Außenfläche jeder flexiblen Klaue ausgesparten Vertiefung (38) aufgenommen ist.

13. Verwendung einer biopharmazeutischen Anordnung auf dem Gebiet der Biopharmazeutik, aufweisend eine Vorrichtung nach Anspruch 12, in welcher die ringförmige Rippe (28) einen Abschnitt mit einer Spitze aufweist, die zum hinteren Ende des Rohrs hin gerichtet ist.

14. Verwendung einer biopharmazeutischen Anordnung auf dem Gebiet der Biopharmazeutik, aufweisend eine Vorrichtung nach einem der Ansprüche 1 bis 13, in welcher der Ring einen Befestigungsclip (47) aufweist, der konfiguriert ist, in der aktiven Position das Einrasten des Rings an dem Kragen (16) sicherzustellen.

15. Verwendung einer biopharmazeutischen Anordnung auf dem Gebiet der Biopharmazeutik, aufweisend eine Vorrichtung nach einem der Ansprüche 1 bis 14, in welcher der erste Verbinder oder der Ring außerdem eine Kennung (50) des Typs Strichcode oder RFID-Etikett oder Farbcode aufweist.

16. Verfahren zur Bildung einer biopharmazeutischen Anordnung, die eine Kopplungsvorrichtung aufweist, die vorgesehen ist, ein glattwandiges weiches Rohr (11) für biopharmazeutisches Fluid mit einem rohrförmigen Stück (9) eines Verbinders (1) auf dichte Weise zu koppeln,
wobei das Verfahren die folgenden Schritte aufweist:
/a/ Bereitstellen eines Verbinders aus geformtem Kunststoff, wobei der Verbinder aufweist:
* ein rohrförmige Stück (9), auf das ein Ende eines glattwandigen weichen Rohrs gemäß einer Achse A geschoben werden kann und das eine Anschlagzone (30) hat, um den Vorschubweg des Rohrs zu begrenzen,
* mehrere flexible Klauen (3), die sich von einer Mittelzone (7) des Körpers des Verbinders in Richtung freies Ende (1a) des Stücks erstrecken, wobei die flexiblen Klauen um den gesamten Umfang des Stücks verteilt sind und mit der Mittelzone (7) des Körpers integral sind und sich in ihrer Ruheposition gemäß einem Kegel erstrecken, der sich zum Ende (1a) des Stücks hin öffnet,
* eine Verbindungsschnittstelle (6; 21), die auf der bezüglich des rohrförmigen Stücks entgegengesetzten Seite angeordnet ist,
/b/ Bereitstellen eines separaten Rings (2) und sein Anordnen in einer Warteposition hinten am Rohr,
/c/ Schieben des glattwandigen weichen Rohrs (11) auf das rohrförmige Stück bis zu der Anschlagzone,
/d/ axiales Verschieben des Rings aus der Warteposition in Richtung Körper des Verbinders bis in eine aktive Position, wobei der Ring geeignet und vorgesehen ist, in der aktiven Position die flexiblen Klauen (3) zu bedecken, um sie radial einwärts zu zwängen, um das Rohr an das Stück zu pressen, wobei die Klauen sich im Wesentlichen parallel zur Achse (A) erstrecken, wenn der Ring in der aktiven Position ist.

## Claims

1. Use in the biopharmaceutical field of a biopharmaceutical assembly comprising a coupling device adapted and intended for coupling a first wall defining a first fluid space (71), in the form of a flexible pipe (11), to a second wall formed by a tubular nozzle (9) of a connector (1), in a biopharmaceutical assembly, to enable establishing a seal between the first wall and the second wall formed by the tubular nozzle, the coupling device comprising:
- said connector (1) with:
. said tubular nozzle (9), onto which one end of the pipe can be inserted along an axis A, from a first end of the nozzle until it reaches a stop area (30) for the pipe,
. a plurality of flexible tabs (3) extending from an intermediate area (7) of the connector body toward the free end (1a) of the nozzle, the flexible tabs being distributed all around the circumference of the nozzle,
. a connection interface (6; 21) arranged opposite the tubular nozzle,
- a separate ring (2) which is axially movable between an inoperative position and an operative position, adapted and intended for covering the flexible tabs (3) so as to force them radially inward in a manner that presses the pipe onto the nozzle in said operative position, thereby creating a radial sealing contact between the pipe and the tubular nozzle,
the tabs extending substantially parallel to the axis (A) when the ring (2) is in the operative position, wherein the separate ring is moved in the direction of the connector body to transition from the inoperative position to the operative position,
and wherein the flexible tabs (3) are formed integrally from the intermediate area (7) of the body, and when at rest they lie in a cone which opens towards the end (1a) of the nozzle, such that the end of the flexible pipe can be inserted onto the nozzle without the flexible tabs posing an obstacle.

2. Use in the biopharmaceutical field of a biopharmaceutical assembly comprising a device according to claim 1, wherein the free ends (32) of the flexible tabs comprise chamfers (33) for the ring.

3. Use in the biopharmaceutical field of a biopharmaceutical assembly comprising a device according to one of claims 1-2, wherein the ring comprises a conical flared region (29) which slides over the free ends of the flexible tabs (3).

4. Use in the biopharmaceutical field of a biopharmaceutical assembly comprising a device according to one of claims 1-3, wherein the connector comprises a collar (16) and the collar forms a stop to end the axial insertion movement of the ring.

5. Use in the biopharmaceutical field of a biopharmaceutical assembly comprising a device according to one of claims 1-4, wherein the flexible tabs comprise at least one tooth (31) on the radially inner face, such that the pressure on the pipe is greater at this tooth.

6. Use in the biopharmaceutical field of a biopharmaceutical assembly comprising a device according to claim 5, wherein the nozzle comprises a notch (19) that is axially offset relative to the tooth (31).

7. Use in the biopharmaceutical field of a biopharmaceutical assembly comprising a device according to one of claims 1-6, wherein, in the operative position of the ring, the tabs (3) are sandwiched between the ring and the pipe for a length (L1) representing more than half of the axial length of the ring, and preferably for a length greater than the inside diameter (D1) of the nozzle.

8. Use in the biopharmaceutical field of a biopharmaceutical assembly comprising a device according to one of claims 1-7, wherein, in the operative position of the ring, the flexible tabs (3) are adjacent to one another in the circumferential direction.

9. Use in the biopharmaceutical field of a biopharmaceutical assembly comprising a device according to one of claims 1-8, wherein the first connector (1) forms a male interface (6) adapted to be received in a second connector (20) forming a female interface.

10. Use in the biopharmaceutical field of a biopharmaceutical assembly comprising a device according to one of claims 1-9, wherein the first connector comprises an attachment disc (21) configured to be attached directly to a filter or bag type of container.

11. Use in the biopharmaceutical field of a biopharmaceutical assembly comprising a device according to one of claims 1-10, wherein the ring (2) comprises a radially external shoulder (26) to facilitate the movement from the inoperative position to the operative position.

12. Use in the biopharmaceutical field of a biopharmaceutical assembly comprising a device according to one of claims 1-11, wherein the ring (2) comprises an annular rib (28) received in a notch (38) provided on the outer face of each flexible tab.

13. Use in the biopharmaceutical field of a biopharmaceutical assembly comprising a device according to claim 12, wherein the annular rib (28) has a cross-section with a tip pointing towards the rear of the pipe.

14. Use in the biopharmaceutical field of a biopharmaceutical assembly comprising a device according to one of claims 1-13, wherein the ring comprises a fastening clip (47) configured to clip the ring onto the collar (16) in the operative position.

15. Use in the biopharmaceutical field of a biopharmaceutical assembly comprising a device according to one of claims 1-14, wherein the first connector or the ring further comprises an identifier (50) such as a barcode or RFID tag or color code.

16. Method for forming a biopharmaceutical assembly comprising a coupling device intended for sealingly coupling a flexible pipe (11) having a smooth wall for biopharmaceutical fluid to a tubular nozzle (9) of a connector (1),
the method comprising the steps of:
**/a/** providing a connector of molded plastic, said connector comprising:
. a tubular nozzle (9), onto which one end of a flexible pipe having a smooth wall can be inserted along an axis A, with a stop area (30) to end the insertion path of the pipe,
. a plurality of flexible tabs (3) extending from an intermediate area (7) of the connector body toward the free end (1a) of the nozzle, the flexible tabs being distributed all around the circumference of the nozzle, and formed integrally from the intermediate area (7) of the body, and when at rest extending in a cone which opens toward the end (1a) of the nozzle,
. a connection interface (6; 21) arranged opposite the tubular nozzle,
**/b/** providing a separate ring (2), and placing it in an inoperative position at the rear of the pipe,
**/c/** inserting the flexible pipe (11) having a smooth wall onto the tubular nozzle until the stop area is reached,
**/d/** moving the ring axially from the inoperative position toward the connector body into an operative position which is adapted and intended for covering the flexible tabs (3) so as to force them radially inward in a manner that presses the pipe onto the nozzle in said operative position, the tabs extending substantially parallel to the axis (A) when the ring (2) is in the operative position.
